# EUROPEAN PATENT SPECIFICATION

(11) **EP 0 339 656 B1**
(45) Date of publication and mention of the grant of the patent: **02.11.1994**
(21) Application number: 89107697.8
(22) Date of filing: 28.04.1989
(51) Int. Cl.: A61K 35/20, A23J 1/20

(54) **Undenatured whey protein concentrate**
Undenaturiertes Proteinkonzentrat aus Molke
Concentré des protéins de petit-lait non-denaturées

(30) Priority: 29.04.1988 US 188271; 23.12.1988 US 289971
(43) Date of publication of application: 02.11.1989
(73) Proprietor: IMMUNOTEC RESEARCH CORPORATION LTD., Montreal, Quebec H3A 2R7 (CA)
(72) Inventor: Gold, Phil, Prof., Westmount, Quebec H3Y 2S4 (CA); Bounous, Gustavo, Montreal, Quebec H3G 1A7 (CA); Kongshavn, Patricia L., St. Lambert, Quebec J4P 2S2 (CA)
(74) Representative: Körber, Wolfhart, Dr.rer.nat.

(56) References cited:
- EP-A- 0 022 696
- EP-A- 0 239 722
- WO-A-87/04050
- FR-A- 2 296 428
- GB-A- 1 495 940
- US-A- 4 710 387
- US-A- 4 753 926
- US-A- 4 784 685
- M.K. Horwitt, The Vitamins, 2. ed., vol.V,1972, 88-96

## Description

The present invention relates to the use of whey protein concentrate obtained front milk, in which the proteins are in their undenatured state for producing a drug containing such whey protein concentrate in accordance with the claims and is based on the effect of dietary whey protein of increasing cellular glutathion levels.

It was namely found with surprise that a better effect can be achieved with the therapeutical use of whey protein concentrate according to the invention, i.e. the whey concentrate obtained from milk and containing all major whey proteins in undenatured state in the scope or field discussed in more detail below than in cases of prior therapeutical applications of undenatured protein fractions of whey (cf. eg. EP-A-022696 and EP-A-0239722). In particular the present invention shows and teaches that further fractionation lowers the effect on glutathione levels.

Whey, and whey protein have been utilized from time immemorial for nutritional purposes. In addition, whey was recommended in folk and ancient medicine for the treatment of various diseases and, in one instance, lifetime feeding of hamsters with a whey protein diet has been shown to promote longevity with no explanation given. We have shown, in controlled experiments, for the first time, that whey protein feeding specifically enhances mice immune response to sheep red blood cells (SRBC), resistance to pneumococcal infection, inhibits the development of colon cancer and delays the process of aging independently of its nutritional quality.

The search for the possible mechanism of immunoenhancement by whey protein feeding has revealed to us the provocative possibility that whey protein may contribute to a broader biological effect of a protective nature involving susceptibility to cancer, diseases of aging and general detoxification of environmental agents. All these conditions appear to be somehow related to changes in glutathione which is a ubiquitous element exerting a protective effect against superoxide radicals and other toxic agents. Our studies have shown that the observed enhancement of the immune response is associated with greater production of splenic glutathione in immunized mice fed whey protein in comparison to mice fed a casein or cysteine enriched casein diet. The efficiency of dietary cysteine in inducing supernormal glutathione levels is greater when it is delivered in the whey protein than as free cysteine. Glutathione was found at higher levels in the heart end liver of whey protein fed old mice in comparison to mice fad the corresponding casein diet or Purina Mouse Chow. In old mice dietary whey protein was found to delay the onset of the diseases of aging.

The Definitions being used in this case are:
(a) Whey Protein:
   Whey proteins are the group of milk proteins that remain soluble in "milk serum" or whey after precipitation of caseins at ph 4.6 and 20°C. The major whey proteins in cow's milk are beta-lactoglobulin (βL), alpha-lactalbumin (αL), immunoglobulin end serum albumin (SA) in order of decreasing amounts. (7)
   The product of industrial separation of this protein mixture from whey is called "whey protein concentrate" (W.P.C.) or isolate. The WPC used in our experiments is from bovine milk (LACPRODAN-80 from DANMARK PROTEIN A.S).
(b) C = casein;
(c) SRBC = Sheep red blood cells;
(d) PFC Plaque forming cells (spleen):
   enumeration of PFC in spleen is used to assess the humoral immune response to SRBC injection;
(e) GSH Glutathione( γ-glutamyl-cysteinyl-glycine); and
(f) Unless otherwise specified, the defined formula diets tested varied only in the type of protein.
(g) Whey of bovine milk contains approximately 6 g per litre protein, most of the lactose, mineral and water soluble vitamins.

A suitable source of whey protein concentrate is the material known by the trade mark PROMOD, which is a protein supplement provided in powder form by Rose Laboratories, a Division of Abbott Laboratories U.S.A. This is a concentrated source of high quality protein which is useful for providing extra protein to persons having increased protein needs, or those who are unable to meet their protein needs with their normal diet. It contains whey protein concentrate and soy lecithin. It has the following nutrients:

| NUTRIENTS: | Per 5 g Protein (one scoop) |
|---|---|
| Protein 5.0 g | |
| Fat | Does not exceed 0.60 g |
| Carbohydrate | Does not exceed 0.67 g |
| Water | Does not exceed 0.60 g |
| Calcium | Does not exceed 23 mg (1.15 mEq) |
| Sodium | Does not exceed 13 mg (0.57 mEq) |
| Potassium | Does not exceed 65 mg (1.66 mEq) |
| Phosphorus | Does not exceed 22 mg |
| Calories | 28 |

It has the following typical amino acid composition per 100 g protein, 100 g PROMOD protein yields approximately 105 g of aminoacids.

### TYPICAL AMINO ACID COMPOSITION Per 100 g Protein Essential

- Amino Acids:: Histidine, 1.9 g;
Isoleucine, 6.2 g;
Leucine, 10.8 g;
Lycine, 9.3 g;
Methionine, 2.2 g;
Phenylalanine, 3.6 g;
Threonine, 7.3 g;
Tryptophan, 1.9 g;
Valine, 6.0 g.
- Non-Essential Amino Acids:: Alanine, 5.3 g;
Arginine, 2.6 g;
Aspartic Acid, 11.2 g;
Cysteine, 2.6 g;
Glutamic Acid, 18.2 g;
Glycine, 2.1 g;
Proline, 6.5 g;
Serine, 5.6 g;
Tyrosine, 3.4 g.

The concentration of some vitamins in the defined formula diet used in (most of) our experiments is given in, Table 1 (Diet 1). Diets are prepared in the following way: 20 g of selected pure protein, 56 g of product 80056 protein free diet powder (Mead-Johnson Co. Inc., U.S.A.), 18 g cornstarch, 2 g wheat bran; 0.05 g Nutramigen vit-iron premix (Bristol-Myers, Ontario, Canada), 2.65 g kCl, 0.84 g NaCl. Unless otherwise specified, the only variable in the various purified diets was the type of protein.

Dairy products ore widely used is a good source of nutrition. In addition claims have been made to the effect that fermented whole milk (yogurt) is beneficial in the management of some types of intestinal infections. Certain dietary regimen based on ill defined cultured dairy products are said to be associated with long life expectancy in some regions of the USRR (Georgia etc).

Since time immemorable, serum lactis latin for milk serum or whey, has been administered to the sick for the treatment of numerous ailments. In 1603 Baricelli(7) reported on the therapeutic use of cow or goat silk serum, sometimes mixed with honey or herbs. The spectrum of illness treated with whey included jaundice, infected lesions of the skin, those of the genito-urinary tract with purulent secretions, gonorrhea, epilepsy, quartan fever and other febrile states of different origins. Indeed, the common denominator of moot of these illnesses appears to be a septic condition. Although physicians of both Ancient Times and of the Middle Ages agreed that whey treatment should be carried out over a period of several days, a difference of opinion appeared to exist concerning the daily amount prescribed. Thus, Galen, Hippocraces and Dioscoride insisted on a minimum daily amount of two latin libras C = 2 x 12 latin uncias = 654,9 g), and up to five libras a day according to gastric tolerance. This would represent between 1 to 2 liters of whey a day. Baricelli, on the other hand, reflecting the trend of his times, limited the amount prescribed to one libra a day, given in fractionated doses on on empty stomach.

In the following year, Costaei(7) wrote about the virtues of whey in the treatment of several unrelated syndromes including broncopneumonitis and diarrhea with high fever. Unfortunately, in his long dissertation, the author fails to clearly discriminate between milk and milk serum treatment.

Since then, numerous articles published in Europe throughout the 17th, 18th and 19th centuries have advocated the therapeutic use of whey. In an Italian textbook published in the middle of the 19th century, at the dawn of scientific medicine, an interesting distinction is made between whole milk and milk serum. Milk is recommended firstly as a nutrient especially in patients with strictures of the gastrointestinal tract. In this respect the author emphasizes that the benefits of the then popular "milk therapy" of cachexia and tuberculosis are due only to the nutritional property of milk. Secondly, milk was prescribed in the treatment of poisoning because milk components would presumably neutralize ingested toxic material. Thirdly, milk therapy was suggested for the alleged capacity of this fluid to coat and sooth ulcers of the gastrointestinal tract.

Milk serum, on the other hand, was advocated in treatment of pneumonitis, acute inflammatory diseases of the intestines and urogenital tract, in spite of its recognized lower nutritional quality. Finally the author emphasized the ineffectiveness of whey in the treatment of disorders of the nervous system. (7)

The prime difference between whey, (serum lactis) and whole milk is the near absence in the former of the caseins, the casein-bound calcium and phosphate, most of the fat and fat soluble vitamins. The Actual concentration in whey of "whey proteins" is only about 5% higher than that in milk. Hence quantitative differences between whey and milk could no: be construed to represent a key factor in the alleged therapeutic effect of whey treatment because, if any, they imply the lack, in whey, of some important nutrients. Our data (7,14) may provide a scientific background to the presumed benefit of intensive treatment with "serum lactis".

We have shown the importance of the characteristic amino acid profile of whey protein concentrate in the immune enhancing effect of WPC. The caseins represent 80% of the total protein content of cow's milk while WPC is only 20%. Hence, it is conceivable that it is the separation of WPC from the caseins in whey which represents the crucial qualitative change, since this will render the amino acid profile of whey proteins unaltered by that of the caseins, once the digestive process has released free amino acids from all ingested proteins. (7)

Although no clinical trials have been reported, some interesting information can be gathered from data on human subjects.

Infant feeding studies indicate that whey predominant formulas are metabolically superior to casein predominant formulas (8, 9) in preterm babies.

Studies performed at the Eppley Cancer Center in Nebraska (10, 11) showed that survival (resistance to spontaneous diseases) of female and male hamsters, measured over a 20 week period of feeding from 4 weeks of age, was best with 20 g/100 g (grams per hundred grams) WPC diet, in comparison with a 20 g/100 g methionine and cysteine supplemented casein diet. Body weight gains were similar in both groups. In lifetime feeding studies, the mean and maximal longevity of female and male hamsters fed 10, 20 and 40 g WPC/100 g diet was increased in comparison with those fed commercial laboratory feed (estimated 24% protein from various sources). Survival was beat with the 20% WPC diet; in males, longevity increased by 50%. No significant relationship was noted between food intake, maximal weight and longevity.

Our interest in the effect of amino acid intake upon the immune system was prompted by an observation made several years ago (1). We fed mice a defined formula diet containing a free amino acid mixture duplicating casein. Another group of mice was fed a similar diet but with moderate restriction of phenylalanine and tyrosine compensated by a corresponding increment in the non-essential amino acid mixture. The second group of mice gained weight at the same rate as the mice fed the casein equivalent mixture or Purina mouse chow. However, when challenged with sheep red blood cells, these mice produced wore antibodies and plaque forming cells against sheep red blood cells than Purina or casein equivalent-fed mice.

A new concept thus emerged, namely, that changes in the amino acid profile of the diet can influence the immune response independently of any systemic effect on the nutritional status of the host. But, more importantly, changes in the amino acid profile, i.e. protein type, could conceivably enhance the humoral immune response beyond that which had traditionally been considered to represent a "normal" response.

We subsequently assessed the effect on the immune response of different types of proteins in nutritionally adequate diets. Mice fed formula diets containing 20% or 28% whey protein concentrate (WPC) were found to produce more plaque forming cells to sheep red blood cells than mice fed Purina mouse chow containing about 22% protein from various sources and of similar nutritional efficiency. The immune enhancing effect of whey protein was maximal at 20% concentration (2). A 20 g net protein/100 g diet provides a good method to asses the effect of protein type on the immune system. At this level most protein supplies the minimum daily requirement of all indispensible amino acids for the growing mouse and this is important because the amino acid distribution, and not adequacy, is the variable under investigation.

In subsequent studies we have compared the effect of dietary whey protein to that of other purified proteins in formula diets of similar nutritional efficiency. The effect of graded amounts of dietary whey protein (L), casein (C), soy (S), wheat (W) protein and Purina rodent chow (stock diet) on the immune responsiveness of C3H/HeN mice has been investigated by measuring the specific humoral immune response to sheep red blood cells (SRBC), and horse red blood cells (HRBC). The nutritional efficiency of those diets was normal and similar.

The immune response of mice fed the WPC diets, was found to be almost five times higher than that of mice fed the corresponding C diets. The humoral immune response of mice fed C, S, and W diets was substantially lower than that of mice fed stock diet, whereas that of mice fed L diet was higher. The above-described immune effect of all tested proteins was obtained at 20 g/100 g concentration with no further increments with 30- and 40 g/100 g protein in the diet.(4)

Because L [whey protein (w.p.)] was tested in comparison to a limited number of proteins, we could not ascertain at that time whether the enhancement of the humoral immune response observed in five (5) unrelated strains of mice fed a whey protein diet, was due to a real immunoenhancement, in absolute terms, by whey protein feeding or immuno-depression by the other food protein tested.

Indeed, we can now state, in retrospect, that these few purified food proteins (casein, soy and wheat) used as "control" for the whey protein mixture were immunosuppressive when compared to all of the other purified food protein subsequently tested, though nutritionally adequate and similar at 20% concentration in diet.

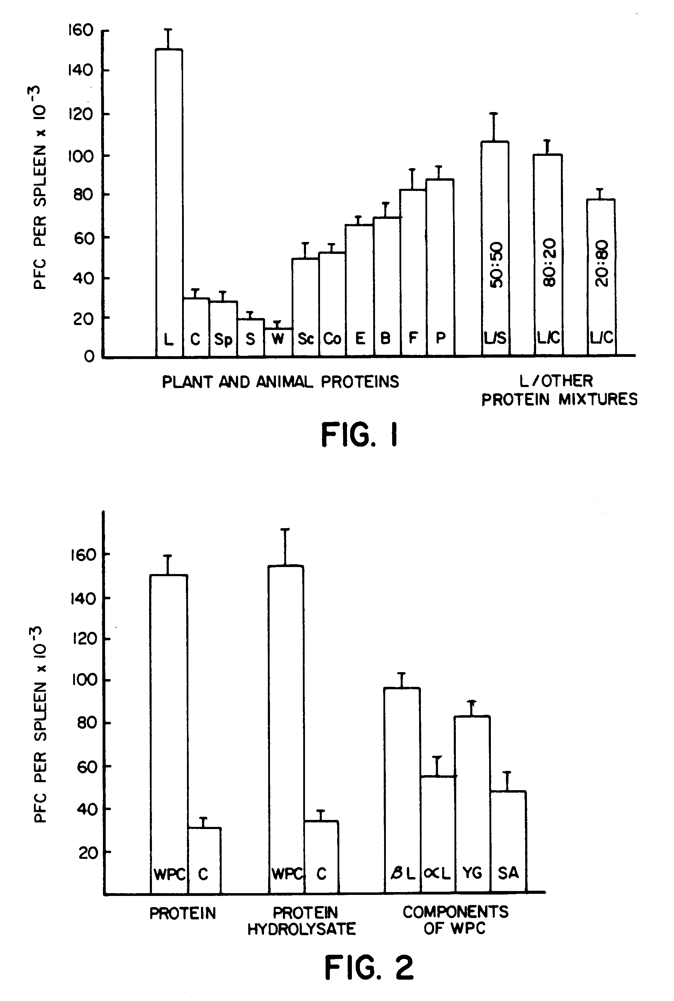

In fact, we subsequently tested whey protein against most commercially available purified food protein (casein, soy, wheat, corn, egg white, beef, fish protein, gamma globulin, beta-lactoglobulin, alpha-lactalbumin, serum albumin, spirulina maxima or scenedesmus algae protein) and found that indeed mice fed whey protein exhibit the highest immune response to foreign antigen (SRBC) (12) (Figure 1). These proteins are nutritionally similar and adequate at the 20 g/100 g diet concentration (Table 2).

We have concluded that our newly discovered immune enhancing and host resistance promoting property of whey protein which we wish to protect by patent is not related to the already known nutritional quality of this protein. In fact, the nutritional property of whey protein at 20 g protein per 100 g diet concentration as used in our experiment is similar to that of the other proteins tested.

In the drawings which form a part of this specification, Figs. 1 and 2 show plaque forming cells-spleen (PFC) on the day showing peak production of pfc following immunization with SRBC.

Fig. 3 shows increased immune response noted with a 28% whey protein diet.

Fig. 4 shows the effects of whey protein on GSH, following immunization with SRBC (14).

Fig. 5 and Fig. 6, respectively, show the heart and liver GSH levels which were observed in old C-57 BL/GNlA mice fed the whey protein (WP) diet in comparison with mice fed the equivalent C diet or Purina Mouse Chow.

Fig. 7 illustrates survival curves showing the relative mortality of mice fed Purina laboratory chow, casein and whey during a seven month period.

Fig. 8 shows the effect of 26 days dietary treatment on plaque forming cells (PFC) response to sheep red blood cells (SRBC) in mice fed diets with various levels of vitamines as indicated in Table 1.

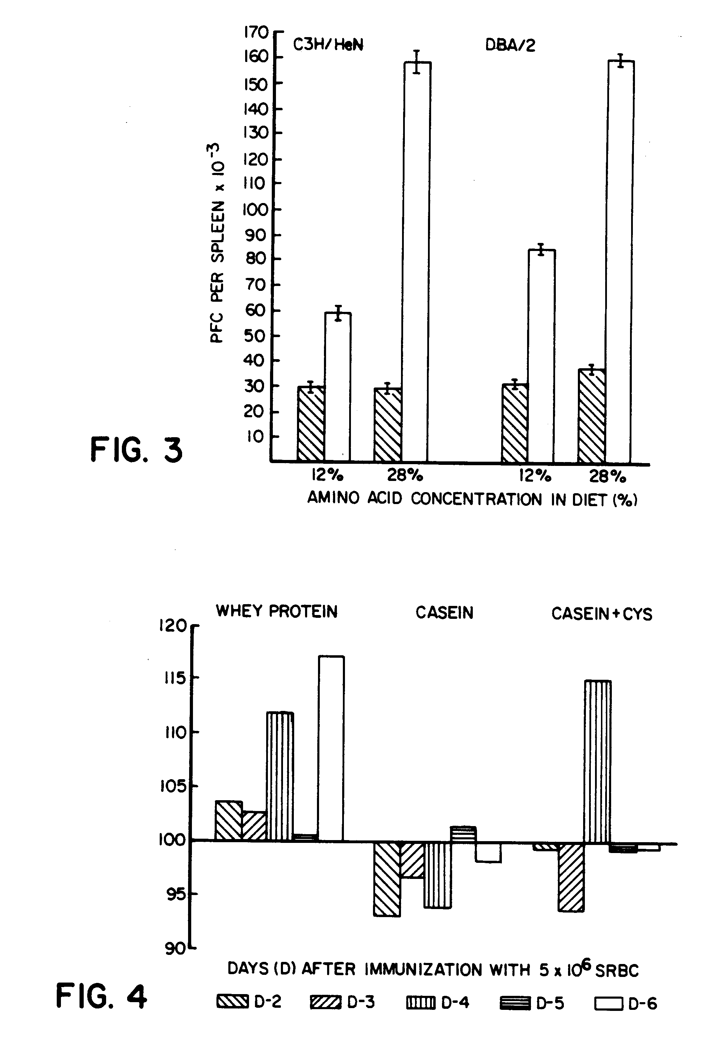

As shown in reference 3 of the attached list of references showing current and previous work, it can be demonstrated that although no significant differences in body growth are seen between the 12% whey protein and 28% whey protein diets, a dramatic enhancement of the immune response is noted with the 28% whey protein diet, unlike what happens with the casein diet where increasing the protein content from 12% to 28% influences neither body growth nor the immune response. In fact, we have found that the improved results appear to reach a plateau at about 20%. Fig.3 of the drawings illustrates the effect. In Fig.3 there to shown the effect of 3 weeks of dietary treatment with whey protein hydrolysate (open bars) or casein hydrolysate (hatched bars) on the number of plaque-forming cells (PFC) per spleen 5 days after immunization with 5 x 10⁶ sheep red blood cells. Each value represents the mean of 10 mice ± SEM. 28% L diet vs. 12% L diet: P ≦ 0.01 by Student's t-test. By the two-way analysis of variance (F test) for both strain of mice, the effect of the quality of protein (L vs. C) is highly significant, P ≦ 0.001.

As shown in reference 12 on the list, it is clear that despite great differences in immune response to SRBC no difference is seen in food consumption, final weight, and serum proteins among mice fed the various purified proteins at 20 g/ 100 g diet concentration. (See Table 2).

In Table 2, in reference 3, data are presented on the nutritional efficiency of the different diets. Mice fed Purina chow and the 12 or 28% C and L diets increased in body weight by approximately the same amount with similar food consumption ranging from 3.5 g to 3,8 g/24 hours. No significant differences were observed between dietary groups in serum protein values and white cell counts (data not shown).

We made the interesting observations that the delayed occurrence of spontaneous death in mice fed the whey protein diet in comparison to those fed the casein diet or Purina (Table 7), is not associated with any difference in food consumption or body weight among the three dietary groups (Table 8).

Fig. 1 shows plaque forming cells-spleen (PFC) on the day showing peak production of PFC following immunization with 10⁶ SRBC. Effect of 2 weeks of dietary treatment with 20 g/100 g diet of either whey protein (L), casein (C), Spirulina maxima protein (Sp), soy protein (S), wheat protein (W), Scenedesmus protein (Sc), corn (Co) protein, egg albumin (E), beef protein (B), fish protein (F), Purina mouse chow (P), or 20 g/100 g diet of a mixture containing 50% L, and 50% S (L/S), or 80% L and 20% C, or 20% L and 80% C (L/C). Each value represents the mean ± SD. See text (reference 12) for statistical significance of differences. L=whey protein concentrate.

Fig. 2 shows plaque forming cells/spleen (PFC) on day showing peak production of PFC following immunization with 10⁶ SRBC. Effect of 3 weeks of dietary treatment with 20 g/100 g diet of either whey protein concentrate (WPC), casein (C), whey protein concentrate hydrolysate, casein hydrolysate, beta-lactoglobulin (βL), alpha-lactalbumin (αL), gamma-globulin (γG) or bovine serum albumin (SA). Each value represents the Mean ± SD.

Our studies show that the immunoenhancing effect of WPC in comparison to C is maintained when these two proteins are replaced in formula diets by a pancreatic hydrolysate (free amino acid and oligo peptides with MW less than 1000) (Figure 2) (7,12). Our results also indicate that mice fed diets containing any one of the four major protein components of the WPC mixture developed a PFC response to SRBC inferior to that of mice fed the corresponding whey protein mixture. We can thus conclude that the observed immunoenhancing effect of WPC is dependent upon the contribution of all its protein components.

For those reasons we can assume that this phenomenon is not related to milk protein allergy or some other manifestation of oral immunization. (14)

Over the past few years we have attempted to identify the changes induced by dietary protein type which might directly or indirectly affect the humoral immune responsiveness. In mice not challenged with an immunogenic stimulus, the type of protein in the diet was found to have little or no effect on a variety of parameters examined. Thus, body growth, food consumption, serum protein, minerals and trace metals, circulating leukocytes and more specifically, the genesis of bone marrow B lymphocytes were all within normal limits (3-7). These findings confirm that at 20 g/100 g diet concentration, the proteins provide an adequate daily supply of essential amino acid for the growing mice. The only significant effect of protein type was found to be in change in plasma amino acid profile, which essentially conformed to the amino acid composition of the ingested protein, with the notable exception of cysteine. (Tables 3, 4).

We were particularly intrigued by the finding that, in spite of an 8-fold higher cysteine content in WPC, the plasma level of cysteine in WPC diet-fed mice was not different from that in their C diet-fed counterparts. The fate of the excess cysteine was a matter of interest. Dietary cysteine is a rate limiting substrate for the synthesis of glutathione (GSH) which is necessary for lymphocyte proliferation. The redox state of the lymphocyte can modulate the intracellular concentration of cyclic GMP, which is known to be intimately involved in lymphocyte proliferation.

Our studies have shown that the observed enhancement of the immune response is associated with greater production of splenic glutathione in immunized mice fed whey protein in comparison to mice fed a casein or cysteine enriched casein diet. The efficiency of dietary cysteine in inducing sypernormal glutathione levels is greater when it is delivered in the whey protein than as free cysteine (14) (see Fig. 4).

The search for the possible mechanism of immunoenhancement by whey protein feeding has revealed to us the provocative possibility that whey protein may contribute to a broader biological effect of a protective nature involving susceptibility to cancer, diseases of aging and general detoxification of environmental agents. All these conditions appear to be somehow related to a drop in glutathione which is a ubiquitous element exerting a protective effect against superoxide radicals and other toxic agents.

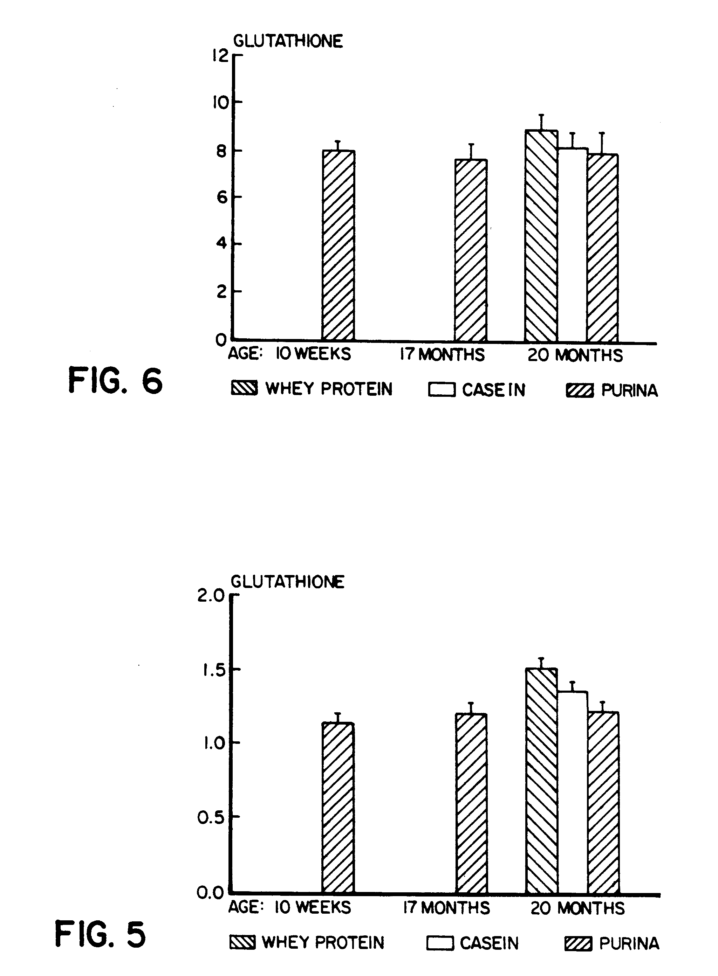

Increased heart and liver GSH levels were observed in old C-57 BL/GNIA mice fed the whey protein (WP) diet in comparison to mice fed the equivalent C diet or Purina Mouse Chow. (Fig.5,6), (reference 16)

In conclusion we have demonstrated that in all the experimental situations above described the immune enhancing effect of whey protein does not depend upon its nutritional property.

Because our studies had shown that dietary protein type influences the humoral immune response, we then proceeded to investigate the effect of WPC in diet on the resistance of mice to pneumococcal infection. Acquired immunity to this infection is largely dependent on the humoral immune response. C3H/He3 mice fed a diet containing 20 g WPC/100 g diet showed improved survival after i.v. infection with Streptococcus pneumoniae type 3 as compared to mice fed a 20 g C/100 g diet of similar nutritional efficiency (7) (Table 5).

On the basis of our various studies, it was shown that the enhanced resistance of mice fed the whey protein diet to infection with Streptococcus pneumoniae type 3 was independent of the weight of the animal at the time of infection and the weight gained before infection (animals were fed the diets for 2 weeks prior to infection). In this connection reference is made to reference 7 on the enclosed list at page 22 and elsewhere.

We have recently observed that a 20 g WPC/100 g diet significantly inhibits the incidence and size of dimethylhydrazine (DMH) induced tumors in the murine colon in comparison to a 20 g C/100 g diet or Purina mouse chow of similar nutritional efficiency. This is a highly immunogenic type of tumors that develop after a long term exposure to the carcinogen. DMH-induced colon tumors appear to be similar to those found in humans as far as type of lesions and chemotherapeutic response characteristics are concerned. The described enhancement of the humoral immune response (heterologous erythrocytes) in WPC fed mice substantiate an immunological mechanism for the observed resistance of WPC fed mice to this immunogenic type of tumor (15) and Table 6.

It should be noted that the similarity of body weight curves among the three dietary groups (whey protein, casein diets and Purina) is consistent with studies in other mouse strains (2-7, 12-14). This effect is striking end appears to rule out conventional nutritional factors for the observed differences in the development of tumors. Reference is made to reference 15 on the enclosed list.

Male C57/BL/6NlA mice were fed ad libitum either 20 g whey protein/100 g diet, or 20 g casein/100 g diet or Purina mouse chow, from age 17 months until sacrificed three months later at age 20 months. GSH (glutathione) content was found to be higher in the liver and heart of whey protein fed mice in comparison to the casein-diet or Purina fed counterparts (Figures 5 and 6) The GSH values in heart and liver of mice fed Purina laboratory chow was almost identical at 17 and 20 months of age. Thus no age related decline is noticed during this period of time. Moreover, the GSH values, at 17 and 20 months of age, of Purina fed mice are similar to those of 10 week old mice. Indeed, the whey protein diet appears to enhance, after 3 months, the GSH content of heart and liver above "normal" values. The mean ± SD body weight changes over the three month period, expressed as percentage of initial weight, of mice fed either the whey protein diet, casein diet or Purina diet was 98.90 ± 17.7, 100.38 ± 15.99 and 99.30 ± 18.50, respectively. Thus, no significant differences were noted in body weight between the various dietary groups. Food consumption was also familiar, varying from 3.4 ± 0.3 g/24 hr in the whey protein diet group to 3.8 ± 0.4 g/24 hr in the Purina fed mice.

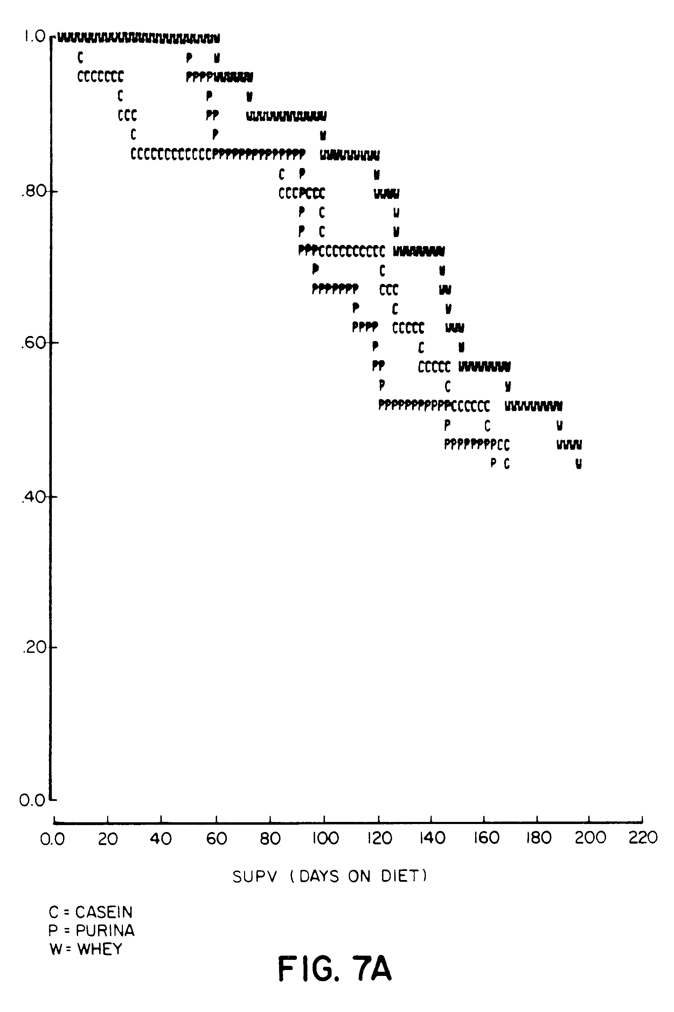

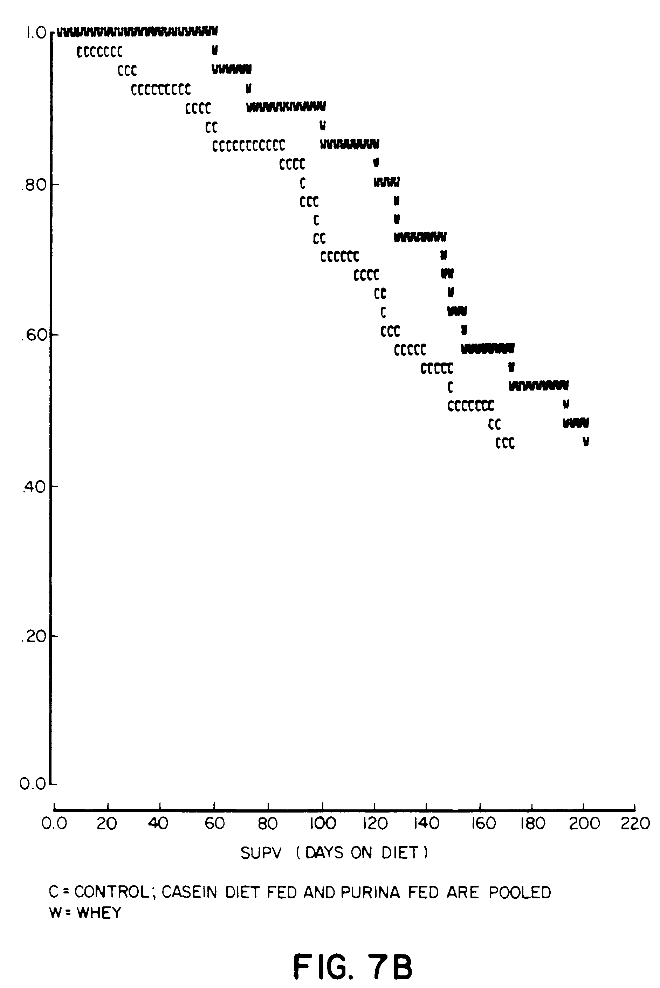

Male C57BL/6NlA mice fed ad libitum at the onset of senescense a 20 g wpc/100 g diet, in pathogen free environment, exhibit delayed mortality in comparison with mice fed Purina laboratory chow over a 7 month observation period extending from the age of 21 months (corresponding human age 55 years) to 28 months of age (corresponding human age to 80 years) at which time 55% mortality is reached. Mean survival time of mice fed the defined formula control diet differing from the whey protein diet only in the type of protein (20 g casein/100 g diet) is almost identical to that of Purina fed controls (Figure 7, Table 7). No significant difference is noticed amongst dietary groups in average body weight changes throughout the experiment (Table 8). Average food consumption in the whey protein-diet group was 2.8 ± 0.4 g/24 hr and 3.0 ± 0.4 g/24 hr in the casein-diet group. The greater amount of spillage of the Purina powder substantially hampers any realistic appraisal of food consumption in this particular group. Whereas other antioxidants, such as Vitamin E, have been shown to be effective primarily in animals that are abnormally deficient in antioxidant synthesis or absorption, dietary whey protein appear to represent an important element in promoting higher tissue GSH levels and in delaying the onset of the diseases of aging in normal wild-type animals. It is important to emphasize that in these longevity studies, the effect of whey protein on the diseases of aging and tissue glutathione is not related to the quality of whey protein as a nutrient which appears to be similar, at 20 g wpc/100 g diet concentration, to that of the other test proteins. A similar conclusion was reached in our previous studies on the effect of a whey protein diet on PFC response to SRBC, resistance to infections and development of tumors,

In section 30 on page 13, page 14 and reference 14 are discussed the effect of whey protein on splenic cell GSH and the relationship between splenic GSH and PFC response to SRBC.

While whey protein represent an optimal source of cysteine, the rate limiting substrate for the biosyntheses of GSH, Vit. B₂ and B₁ are important element in the function of the GSH redox cycle.

Glutathione (GSH) status in tissues is maintained mainly in the reduced state (GSH:GSSG, 250), which is achieved by the efficient GSH peroxidase and reductase system coupled to the NADP+/NADPH redox pair. Endogenous toxic H₂O₂ is reduced to H₂O through the oxidation of GSH to GSSG catalyzed by GSH peroxidase. At the expense of cellular NADPH, GSSG is effectively reduced back to GSH by NADPH;GSSG reductase, thus maintaining thiol balance. As a result, GSSG reductase has a great capacity to protect cells against oxygen toxicity from endogenous active oxygen species.

Vit. B₁ (thiamine) in involved in the transketolase reaction of the pentose phosphate shunt yielding NADPH and pentose.

Vit. B₂ (riboflavin): The coenzyme derivatives of riboflavin, flavin mononucleotide (FMN) and flavin adenin dinucleotide (FAD), are synthesized sequentially from riboflavin. Vit. B₂ deficient animals exhibit marked decreases in activities of FMN and FAD-requiring enzymes, such as GSH reductase.

In this sense it is conceivable that all these water soluble vitamins naturally present in whey, play an essential role for optimal function of the GSH redox cycle particularly when whey protein intake, as shown in our experiments, has produced higher level of GSH synthesis and storage in the tissues.

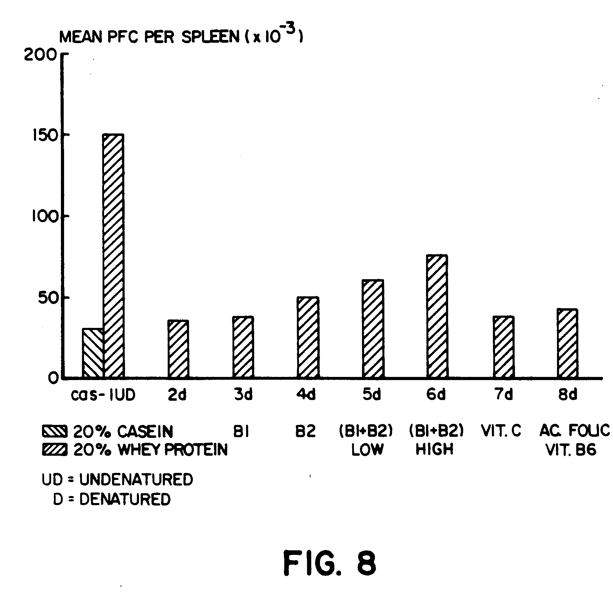

Our studies (Fig. 8) shows that dietary levels of Vit. B₁, B₂ slightly above recommended allowance (Table 1, diets 5,6) significantly contribute to the immunoenhancing effect of dietary whey protein concentrate, Whey protein, by providing optimal bioavailability of the limiting substrate (cysteine) enhances the synthesis and storage of GSH. On the other hand, higher than normal intakes of Vit. B₁ and B₂ are necessary to maintain the GSH redox cycle at a level higher than normal, thus allowing the development of a better than normal immune response to SRBC. Individually the effect of each of the vitamins in whey protein fed mice is limited; however their synergistic effect on the immune response of whey protein fed mice is apparent (fig. 8, diets 5,6 diet 1). The same vitamins are ineffective on the immune response of casein diet-fed mice. Although all these water-soluble vitamins are present in whey, it is interesting to note that the main natural source of the single most effective vitamin, riboflavin, is whey to which Vit. B₂ given its characteristic color.

In conclusion, dietary intake of Vit. B₁ and particularly B₂ above recommended daily allowance contribute to the development of enhanced immune response in whey protein fed animals; Vitamin B₂ + B₁ appears to produce the strongest effect. When intake of these vitamins is at or slightly below these levels, body growth and animal appearance are normal, but the response to immune challenge is below the maximum potential of whey protein fed mice.

In the stomach, whey is separated from milk by the action of gastric juice. It is conceivable that the transit and absorption of the water-soluble vitamins and proteins of whey occur faster than those of the protein (casein) and vitamin constituents of the milk coagulum (curd). Hence the whey protein and vitamins including the vitamins B₁, and B₂ could enter the systemic circulation at a different rate than that of other milk constituents and express their synergistic effect on the immune system and the GSH redox cycle.

The immunoenhancing and the other specific biological properties of dietary whey protein described in this application, are heat labile and dependant upon the undenitured (native) state of the protein (which can also be affected by vigorous shaking, solvents, extreme ph changes, etc.) and are independent of its nutritional quality which is unaltered by the process of denaturation.

Unlike most other commercially available whey protein which are denatured, the whey protein used in our experiments, produced in Denmark (Lacprodan - 80) is 90% undenatured (U.D. in Fig. 8). This protein displays the greatest tendency to denature under heat thus exposing its free sulfhydryl group (17). When experiments were done using a batch of w.p.c. received after a long surface transport from Denmark through the U.S. in exceptionally hot and humid weather (summer 1988), the immunoenhancing property of w.p.c. was lost (Fig. 8, 2d-8d). These experiments, while indicating the synergistic role of vit B, and B2, in the immunoenchancing effect of the diet, also show the negative effect of a presumably partially denatured whey protein. Previous studies have shown (14), that the immunoenhancing property of dietary whey protein is probably related to an optimal intracellular transport and availability of the cysteine which is a limiting precursor for glutathione synthesis. It is conceivable that partial denaturation of this protein had brought about the loss of its specific biological property by altering a cysteine bond crucial for intracellular transport of cysteine and GSH synthesis, without any effect on its nutritional quality.

In conclusion, the preparation of whey protein concentrate made as the "Danmark Protein" lacprodan-80 was produced before 1985 or in a comparable appropriate fashion, produced the biological activity sought. This activity correlates directly with the PFC essay employed in our experiments.

**TABLE 1**

| Vitamin Content of Test Diets | | | | | | | |
|---|---|---|---|---|---|---|---|
| VITAMINS (mg/100g Diet) | REG. (Diet 1) | Diet 3 | Diet 4 | Diet 5 | Diet 6 | Diet 7 | Diet 8 |
| VIT. B1 | 0.34 | 1.42 | | 0.9 | 2.7 | | 1.0 |
| VIT. B2 | 0.38 | | 1.47 | 0.9 | 2.7 | | 0.6 |
| VIT. B6 | 0.26 | | | | | | 0.7 |
| AC. FOLIC | 0.063 | | | | | | 0.1 |
| VIT. C | 53.3 | | | | | 118.3 | |

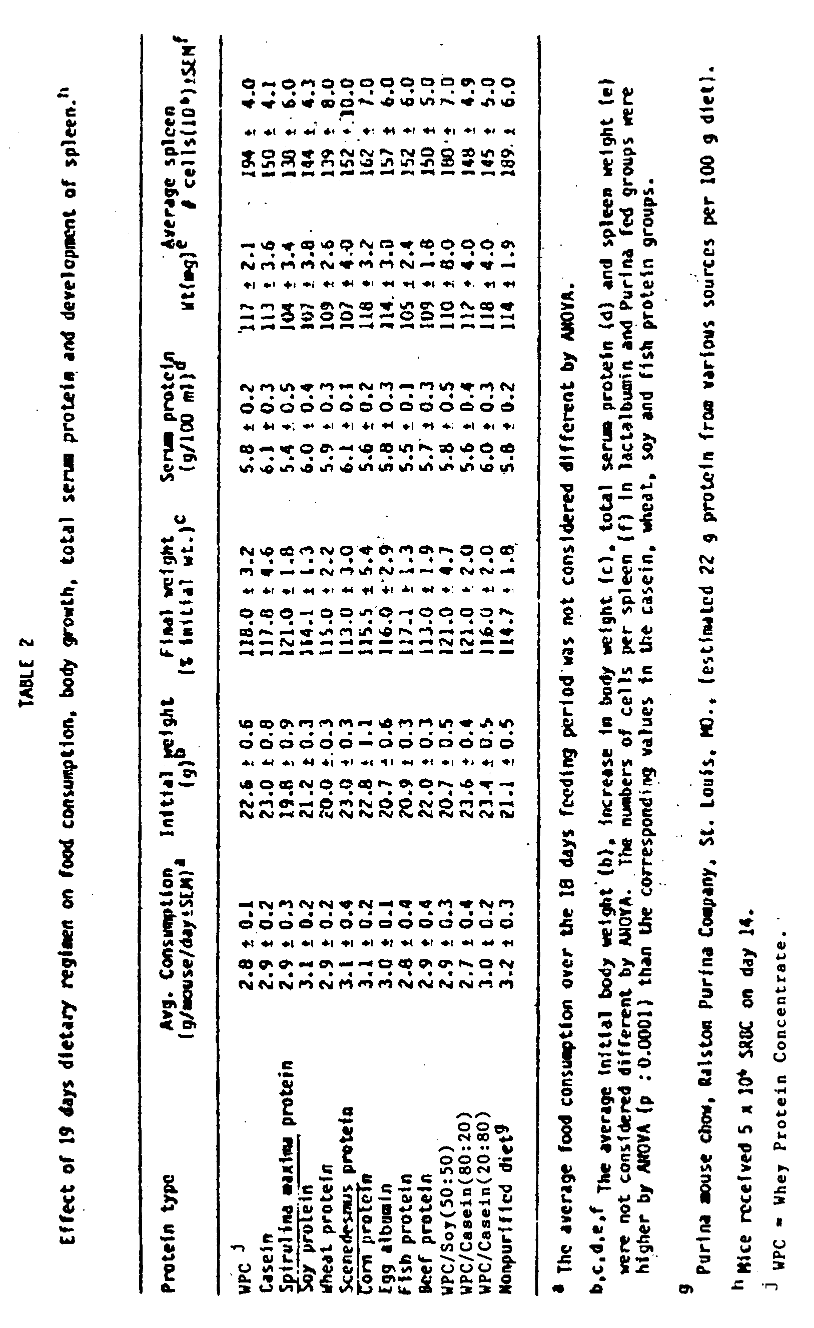

**TABLE 3**

| AMINO ACID COMPOSITION OF TEST PROTEINS ^{(a)} (g/100 g protein) | | |
|---|---|---|
| AMINO ACID | CASEIN | WHEY PROTEIN CONCENTRATE |
| Phenylalanine | 5.3 ± 0.2 | 3.4 ± 0.3 |
| Tryptophan | 1.4 ± 0.2 | 2.1 ± 0.0 |
| Glycine | 2.0 ± 0.1 | 2.0 ± 0.2 |
| Serine | 6.2 ± 0.5 | 5.2 ± 0.4 |
| Leucine | 10.0 ± 0.4 | 10.4 ± 0.7 |
| Isoleucine | 6.0 ± 0.6 | 6.1 ± 0.8 |
| Valine | 7.1 ± 0.3 | 5.8 ± 0.8 |
| Methionine | 2.9 ± 0.2 | 2.1 ± 0.3 |
| Cysteine | 0.3 ± 0.1 | 2.3 ± 0.3 |
| Aspartic acid | 7.3 ± 0.1 | 10.7 ± 0.7 |
| Glutamic acid | 22.9 ± 0.3 | 18.8 ± 0.7 |
| Histidine | 3.0 ± 0.1 | 2.0 ± 0.2 |
| Tyrosine | 6.0 ± 0.1 | 3.0 ± 0.4 |
| Proline | 11.6 ± 0.4 | 6.1 ± 0.7 |
| Arginine | 4.0 ± 0.1 | 2.8 ± 0.3 |
| Alanine | 3.1 ± 0.3 | 4.9 ± 0.4 |
| Lysine | 8.2 ± 0.1 | 9.2 ± 0.5 |
| Threonine | 4.6 ± 0.3 | 6.8 ± 1.3 |

| | | |
|---|---|---|
| ^{(a)} Value expressed as Mean ± S.D. of data from reliable sources (Reference 13). | | |

**TABLE 4**

| EFFECT OF DIETARY PROTEIN TYPE ON PLASMA AMINO ACID PATTERNS | | | |
|---|---|---|---|
| Amino Acid | Whey protein concentrate 20 g% | Casein 20 g% | P-value |
| | nmol/ml | | |
| Isoleucine | 90±5 | 95±8 | - |
| Leucine | 125±5 | 113±4 | - |
| Valine | 232±10 | 278±13 | 0.025 |
| Methionine | 72±3 | 92±6 | 0.025 |
| Cystine | 37±3 | 37±3 | - |
| Phenylalanine | 51±1 | 75±4 | 0.0005 |
| Tyrosine | 55±2 | 83±5 | 0.005 |
| Threonine | 310±7 | 223±2 | 0.0005 |
| Tryptophan | - | - | - |
| Lysine | 301±6 | 323±7 | - |
| Histidine | 50±1 | 64±4 | 0.005 |
| Arginine | 61±4 | 92±6 | 0.005 |
| Glycine | 142±7 | 144±7 | - |
| Serine | 120±8 | 132±4 | - |
| Alanine | 437±18 | 382±19 | 0.05 |
| Proline | 52±5 | 117±10 | 0.0005 |
| Aspartic Acid | 24±2 | 16±1 | 0.005 |
| Glutamic acid | 65±2 | 44±4 | 0,005 |
| Mean ± SD. | | | |

**TABLE 5**

| SUSCEPTIBILITY TO TYPE 3 S. PNEUMONIAE OF THREE SERIES OF MICE FED DIETS OF VARIOUS PROTEIN TYPES¹ | | | | | | |
|---|---|---|---|---|---|---|
| Days Post-Infection² | Experiment 1 | | Ration of alive: dead mice Experiment 2 | | Experiment 3 | |
| | C | L | C | L | C | L |
| 0 (10²) | 8:0 | 8:0 | 10:0 | 10:0 | 10:0 | 10:0 |
| 2 | 8:0 | 8:0 | 10:0 | 10:0 | 10:0 | 10:0 |
| 3 | 7:1 | 8:0 | 10:0 | 10:0 | 10:0 | 10:0 |
| 4 | 7:1 | 8:0 | 9:1 | 10:0 | 9:1 | 10:0 |
| 9 (10³) | 7:1 | 8:0 | 9:1 | 10:0 | 9:1 | 10:0 |
| 11 | 7:1 | 8:0 | 9:1 | 10:0 | 9:1 | 10:0 |
| 12 | 7:1 | 8:0 | 5:5 | 9:1 | 8:2 | 10:0 |
| 13 | 6:2 | 8:0 | 4:6 | 9:1 | 8:2 | 10:0 |
| 14 | 5:3 | 8:0 | 4:6 | 9:1 | 7:3 | 9:1 |
| 40 | 5:3 | 8:0 | 4:6 | 9:1 | 7:3 | 9:1 |

| | | | | | | |
|---|---|---|---|---|---|---|
| ¹ Mice were infected after 2 wk treatment with casein diet (C) (20 g casein/100 g diet), or WPC diet (L) (20 g/100 g). | | | | | | |
| ² Injected i.v. in 1% FCS-Ringer; 9 days after infection with 10² pneumococci the surviving mice were infected with a dose of 10³ pneumococci. C = Casein L = Whey Protein Concentrate. Overall mortality is 36% in the C fed groups and this is significantly higher (P=0.002) than that of the L fed mice which is 7.1%. | | | | | | |

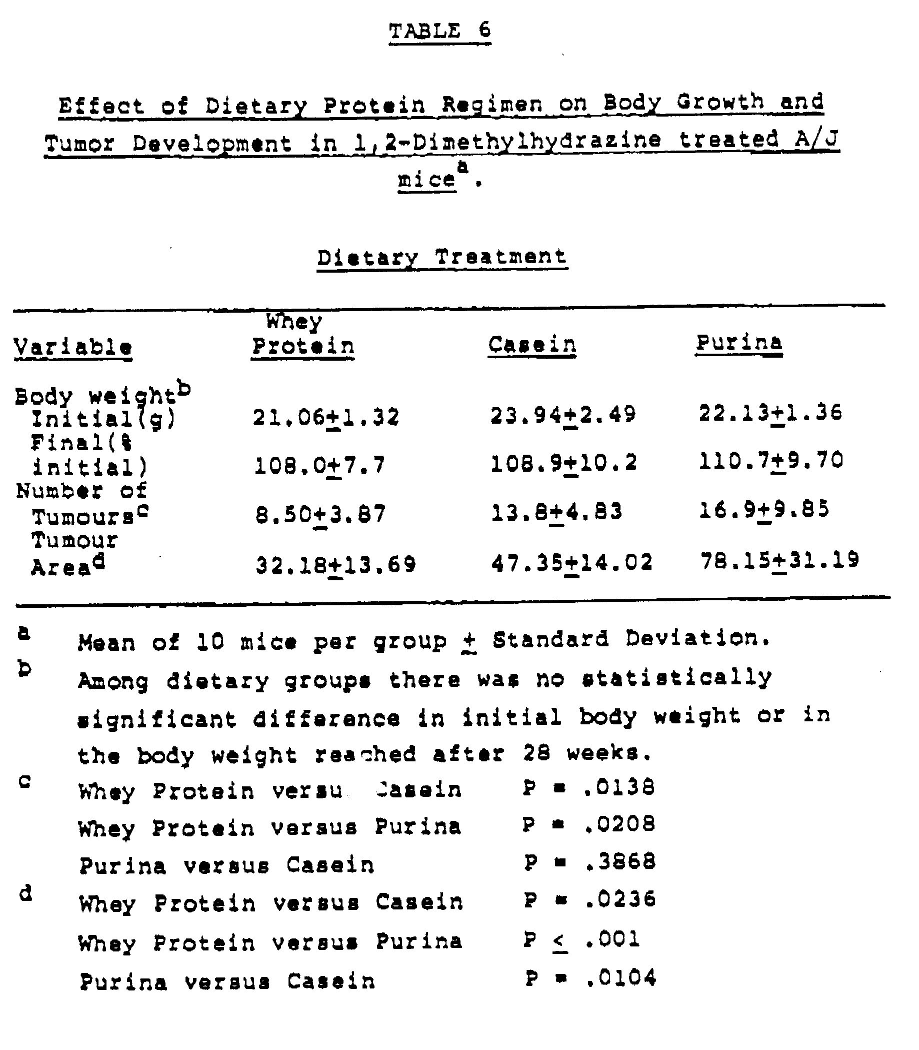

**TABLE 7**

| Time at which 55% of mice fed either of three dietary regimen (from 21 months of age to 28 months of age) were dead. | |
|---|---|
| Dietary treatment | Days of feeding^{(a)} |
| Casein | 92.2 ± 55.2^{(b)} |
| Whey | 125.0 ± 41.6^{(c)} |
| Purina | 92.7 ± 31.7^{(d)} |

| | |
|---|---|
| ^{(a)} Mean of 10 mice per group ± standard deviation. Survival time for d < c (p < 0.05). If the two control diet groups with near identical survival time are pooled together: b,d < c (p < 0.05). | |

**TABLE 8**

| VITAMIN CONTENT/100 g DIET | | | | | |
|---|---|---|---|---|---|
| | Diet 1 | Diet 2 | Diet 3 | Diet 4 | Diet 5 |
| Ascorbic acid (Vitamin C), mg | 65.0 (N)^{a} | 47.0 | 140.0 | 47.0 | 47.0 |
| Niacin, mg | 9.2 | - | - | - | - |
| Riboflavin (vitamin B₂), mg | 0.69 (0.60)^{b} | 0.54 | 0.54 | 1.00 | 0.54 |
| Thiamin (vitamin B₁), mg | 0.63 (0.60)^{b} | 0.54 | 0.45 | 0.45 | 1.00 |
| Folic acid, mg | 0.12 | - | - | - | - |
| Vitamin B₆, mg | 0.36 | - | - | - | - |
| Biotin, mg | 0.058 | - | - | - | - |
| Pantothenic acid, mg | 3.38 | - | - | - | - |
| Choline, mg | 76.0 | - | - | - | - |
| Vitamin B₁₂, mg | 0.55 | - | - | - | - |
| Phylloquinone (vitamin K), mg | 1.8 | - | - | - | - |
| Inositol, mg | 34.39 | - | - | - | - |
| Retinyl palmitate (vitamin A), I.U. | 1800 | - | - | - | - |
| Ergocalciferol (vitamin D₂), I.U. | 360 | - | - | - | - |
| 1-tocopheryl acetate (vitamin E), I.U. | 24.0 | - | - | - | - |
| The mineral content of ions or cations (expressed in milligrams per 100 g diet) and the actual chemical compounds fed are: Ca, 378 (CaHPO₄ . 2H₂O and Ca₃ (C₆H₅O₇)₂ . 4H₂O); P, 208 (K₂HPO₂ . 2H₂O); Fe, 7.7 (FeSO₄ . 2H₂O); Mg, 44 (MgO); Cu, 0.38 (CuSO₄ . 5H₂O); Zn, 2.5 (ZnSO₄ . 7H₂O); Mn, 0.63 (MnSO₄); Cl, 840 (C₅H₁₄CINO); K, 1050 (K₂HPO₄ . 2H₂O); Na, 245 (NaCl). | | | | | |

| | | | | | |
|---|---|---|---|---|---|
| ^{a}N = Not required. | | | | | |
| b = Values between brackettes are the vitamin concentrations of an adequate mouse purified diet. (AIN 76: KNAPKA J. Jr. in "The mouse in biomedical research". Eds. H.L. Foster, J.D. Small, J.G. Fox, Academic Press, New York, p. 58, 1983). | | | | | |

### References Relating to Previous and Current Work

1) Bounous G., Kongshavn P.A.L.
   "The effect of dietary amino acid on immune reactivity" - Immunology 35/257-266/1978
2) Bounous G., Stevenson M.M., Kongshavn P.A.L.
   Influence of dietary lactalbumin hydrolysate on the immune system of mice and resistance to Salmonelloss - I. of Infect Diseases 144/281/1981
3) Bounous G., Kongshavn P.A.L.
   Influence of dietary proteins on the immune system of mice - J. Nutr. 112/1747-1755/1982
4) Bounous G., Letourneau L., Kongshavn P.A.L.
   Influence of dietary protein type on the immune system of mice - J. Nutr. 113/1415-1421/1983
5) Bounous G., Kongshavn P.A.L.
   Differential effect of dietary protein type on the B-Cell and T-Cell immune responses in mice - J. Nutr. 115/1403-1408/1985
6) Bounous G., Shenuda N., Kongshavn P.A.L., Osmond D.G., Mechanism of altered B-Cell response induced by changes in dietary protein type in mice - J. Nutr., 115/1409-1417/1985
7) Bounous G., Kongshavn P.A.L.
   Influence of protein type in nutritionally adequate diets on the development of immunity (In press in) "Absorption and utilization of amino acids" Editor, N. Friedman publisher CRC press Fall 1988.
8) Raiha N.C.R., Heinonen K., Rassin D.K., Gaull G.E. Heindnen,
   Milk protein quantity and quality in low-birth weight infants: 1: Metabolic responses and effects on growth - Pediatric 57/659-674/1976
9) Darling P., Lepage G., Tromblay P,, et al
   Protein quality and quantity in preterm infants receiving the same energy intake.
   Am. J, Dis. Child 139/186-190/1985
10) Birt D.F., Baker P.Y., Hruza D.S.
   Nutritional evaluation of three dietary levels of lactalbumin throughout the lifespan of two generations of syrian hamsters -J. Nutrit 112/2151- 2160/1982
11) Birt D.F., Schuldt G.H., Salmasi S.
   "Survival of hamsters fed graded levels of two protein sources" - Lab Animal Sci 32/363-366/1982
12) Bounous G., Kongshavn P.A.L., Gold P.
   "The immunoenhancing property of dietary whey protein concentrate" "Clinical and Investigat. Med. 11/271-278/1988.
13) Bounous G., Kongshavn P.A.L., Taveroff A., Gold P.
   "Evolutionary traits in human milk proteins" - "medical hypothesis", 27/133-140/1988.
14) Bounous G., Batist G., Gold P.
   Immunoenhancing effect of dietary whey proteins in mice: role of glutathione. Accepted for publication in Clin. Invest. Med.
15) Bounous G., Papenburg R., Kongshavn P.A.L., Gold P, Fleiszer D.
   Dietary whey protein inhibits the development of dimathylhydrazine induce malignancy - Clin./Invert/Med. 11/213-217/1988.
16) Bounous G., Gervais F., Batist G., Gold P.
   Effect of dietary whey protein and tissue glutathione on the diseases of aging. Submitted to "clinical investigat. med."
17) Farrell H.M., Douglas F.W.
   Effect of ultra-high-temperature pasteurization on the functional and nutritional properties of milk proteins. Kieler Milch-wirtschfliche Forach. 35/365-56/1983

## Claims

1. Use of whey protein concentrate obtained from milk in which the proteins are in their undenatured state for producing a drug containing said whey protein concentrate to enhance systemic humoral immune response.

2. Use of whey protein concentrate obtained from milk, in which the proteins are in their undenatured state for producing a drug containing said whey protein concentrate for enhancing resistance to bacterial infection and/or slow growing carcinomas and/or to delay the onset of the diseases of aging, i.e. old age related diseases in mammals and/or animals.

3. Use of whey protein concentrate in which the proteins are in their undenatured state as claimed in claim 1 or 2, wherein the whey protein is bovine whey protein.

4. Use of whey protein concentrate in which the proteins are in their undenatured state as claimed in claim 1 or 2, wherein the whey protein is goat or sheep protein.

5. Use of whey protein concentrate in which the proteins are in their undenatured state as claimed in claim 1 or 2, wherein the whey protein is a mixture of bovine, goat, or sheep whey protein.

6. Use of whey protein concentrate in which the proteins are in their undenatured state as claimed in claim 1 or 2, wherein the whey protein is human whey protein.

7. Use of whey protein concentrate obtained from milk, in which the proteins are in their undenatured state for producing a drug containing said whey protein concentrate for increasing the cellular glutathione levels for enhancement of immune response, detoxification, protection against diseases of aging, and cellular protection against fee radicals, chemical carcinogens and bacterial infections, such as pneumnococcal infections, in mammals and/or animal organs.

8. Use of a combination of a vitamin supplement comprising Vitamin B₂ in an amount in excess of minimum daily requirements, and a therapeutically or prophylactically effective amount of whey protein concentrate obtained from milk in which the proteins are in their undenatured state for producing a drug containing said combination whey for enhancing systemic humoral response in mammals.

9. Use of a combination as claimed in claim 8, wherein said vitamin supplement comprises Vitamins B₁, B₂, in amounts in excess of minimum daily requirements.

10. A dietary supplement for a mammal comprising in combination Vitamins B₁, B₂, in amounts in excess of minimum daily requirements, for that mammal, together with a thereapeutically or prophylactically effective amount of whey protein supplement obtained from milk, in an undenatured state.

## Patentansprüche

1. Verwendung von einem aus Milch erhaltenen Molke-Proteinkonzentrat, in dem sich die Proteine in undenaturiertem Zustand befinden, zur Herstellung von einem dieses Molke-Proteinkonzentrat enthaltendem Arzneimittel zur Erhöhung der systemischen humoralen Immunitätsreation.

2. Verwendung von einem aus Milch erhaltenen Molke-Proteinkonzentrat, in dem sich die Proteine in undenaturiertem Zustand befinden, zur Herstellung von einem dieses Molke-Proteinkonzentrat enthaltendem Arzneimittel zur Erhöhung der Widerstandskraft gegen bakterielle Infektionen und/oder langsam wachsende Carcinome und/oder zur Verzögerung des Eintretens von Alterserkranktungen, d. h. altersbedingten Erkrankungen bei Menschen und/oder Tieren.

3. Verwendung von einem Molke-Proteinkonzentrat, in dem sich die Proteine in undenaturiertem Zustand befinden, gemaß Anspruch 1 oder 2, wobei das Molkeprotein ein Rinderprotein ist.

4. Verwendung von einem Molke-Proteinkonzentrat, in dem sich die Proteine in undenaturiertem Zustand befinden, gemaß Anspruch 1 oder 2, wobei das Molkeprotein ein Ziegenprotein oder Schafprotein ist.

5. Verwendung von einem Molke-Proteinkonzentrat, in dem sich die Proteine in undenaturiertem Zustand befinden, gemaß Anspruch 1 oder 2, wobei das Molkeprotein eine Mischung aus Rinderprotein, Ziegenprotein oder Schafprotein ist.

6. Verwendung von einem Molke-Proteinkonzentrat, in dem sich die Proteine in undenaturiertem Zustand befinden, gemaß Anspruch 1 oder 2, wobei das Molkeprotein ein menschliches Molkeprotein ist.

7. Verwendung von einem aus Milch erhaltenen Molke-Proteinkonzentrat, in dem sich die Proteine in undenaturiertem Zustand befinden, zur Herstellung von einem dieses Molke-Proteinkonzentrat enthaltenden Arzneimittel zur Erhöhung der zellulären Glutathionspiegel für die Verbesserung von Immunitätsreationen, Entgiftungen, Schutz gegen Alterserkrankungen, Schutz der Zellen gegen freie Radikale, chemische Carcinogene und bakterielle Infektionen, wie durch Pneumokokken bedingten Infektionen, in menschlichen und/oder tierischen Organen.

8. Verwendung von einer Kombination von einer Vitaminergänzung, die mehr als den täglichen Minimalbedarf an Vitamin B 2 enthält, mit einer therapeutisch oder prophylaktisch wirksamen Menge an dem aus Milch erhaltenen Molke-Proteinkonzentrat, in dem sich die Proteine in undenaturiertem Zustand befinden, zur Herstellung eines diese Kombination enthaltenden Arzneimittels für die Erhöhung der systemischen humoralen Reaktion bei Säugern.

9. Verwendung der Kombination gemäß Anspruch 8, wobei die Vitaminergänzung die Vitamine B 1, B 2, in über dem täglichen Minimalbedarf liegenden Mengen enthält.

10. Diätergänzung für Säuger, enthaltend eine Kombination der Vitamine B 1, B 2 in über dem täglichen Minimalbedarf für diese liegenden Mengen zusammen mit einer therapeutische oder prophylaktisch wirksamen Menge an dem aus Milch erhaltenden Molkeprotein in undenaturiertem Zustand.

## Revendications

1. Utilisation d'un concentré de protéines du petitlait (lactosérum) obtenu à partir de lait, dans lequel les protéines sont dans leur état non-dénaturé, pour produire un médicament contenant ledit concentré de protéines du lactosérum pour accroître la réponse immunitaire humorale systémique.

2. Utilisation d'un concentré de protéines du lactosérum obtenu à partir de lait, dans lequel les protéines sont dans leur état non-dénaturé, pour produire un médicament contenant ledit concentré de protéines du lactosérum pour accroître la résistance à l'infection bactérienne et/ou aux carcinomes à croissance lente et/ou pour retarder le commencement des maladies du vieillissement, c'est-à-dire des maladies connexes à la vieillesse chez les mammifères et/ou les animaux.

3. Utilisation d'un concentré de protéines du lactosérum dans lequel les protéines sont dans leur état non-dénaturé, telle que revendiquée dans la revendication 1 ou 2, dans laquelle la protéine du lactosérum est une protéine du lactosérum bovin.

4. Utilisation d'un concentré de protéines du lactosérum dans lequel les protéines sont dans leur état non-dénaturé, telle que revendiquée dans la revendication 1 ou 2, dans laquelle la protéine du lactosérum est une protéine du lait de chèvre ou de brebis.

5. Utilisation d'un concentré de protéines du lactosérum dans lequel les protéines sont dans leur état non-dénaturé, telle que revendiquée dans la revendication 1 ou 2, dans laquelle la protéine du lactosérum est un mélange de protéines du lactosérum d'origine bovine, caprine ou ovine.

6. Utilisation d'un concentré de protéines du lactosérum dans lequel les protéines sont dans leur état non-dénaturé, telle que revendiquée dans la revendication 1 ou 2, dans laquelle la protéine du lactosérum est une protéine du lactosérum humain.

7. Utilisation d'un concentré de protéines du lactosérum obtenu à partir de lait, dans lequel les protéines sont dans leur état non-dénaturé, pour produire un médicame contenant ledit concentré de protéines du lactosérum afin d'augmenter les niveaux cellulaires de glutathione pour accroître la réponse immunitaire, pour la désintoxication, pour la protection contre les maladies du vieillissement, et pour la protection cellulaire contre les radicaux libres les carcinogènes chimiques et les infections bactériennes telles que les infections dues au pneumocoque, chez les mammifères et/ou dans les organes des animaux.

8. Utilisation d'une combinaison d'un supplément de vitamines, qui contient de la vitamine B 2 à une quantité supérieure au minimum nécessaire par jour et une quantité thérapeutique ou prophylactique effecive de concentré de protéines du petit-lait (lactosérum) dans lequel les protéines sont dans leur état non-dénaturé pour produire un médicament contenant ladite combinaison pour accroître la réponse immunitaire humorale.

9. Utilisation de la combinaison selon la revendication 8, dans laquelle le supplément de vitamines contient les vitamines B1, B2 à une quantité supérieure au minimum nécessaire par jour.

10. Supplément diétique pour mammifères, comprenant une combinaison des vitamines B 1, B 2 à des quantités supérieures au minimum nécessaire par jour ensemble avec une quantité thérapeutique ou prophylactique effective de concentré de protéines du petit-lait (lacotsérum) obtenu à partir de lait, dans lequel les protéines sont dans leur état non-dénaturé.
